# NEUE EUROPÄISCHE PATENTSCHRIFT

(11) **EP 0 362 704 B2**
(45) Veröffentlichungstag und Bekanntmachung des Hinweises auf die Entscheidung über den Einspruch: **07.08.1996**
(45) Hinweis auf die Patenterteilung: 12.05.1993
(21) Anmeldenummer: 89117998.8
(22) Anmeldetag: 28.09.1989
(51) Int. Cl.: C07C 311/59, C07C 303/42, A61K 31/18

(54) **Verfahren zur Mikronisierung von Glibenclamid**
Process for the micronisation of glibenclamide
Procédé pour la micronisation du glibenclamide

(30) Priorität: 01.10.1988 DE 3833439
(43) Veröffentlichungstag der Anmeldung: 11.04.1990
(73) Patentinhaber: HOECHST AKTIENGESELLSCHAFT, 65926 Frankfurt am Main (DE)
(72) Erfinder: Jaenicke, Ottmar, Dr., D-6233 Kelkheim(Taunus) (DE)

(56) Entgegenhaltungen:
- DD-A- 91 647
- DD-A- 105 215
- DD-A- 204 915
- DD-A- 248 115
- DE-B- 1 301 812
- DE-B- 2 144 303
- FR-A- 2 204 425
- GB-A- 1 420 907
- CHEMICAL ABSTRACTS, Band 107, Nr. 4, 27. Juli 1987, Columbus, Ohio, USA; S. CILIANU-BIBIAN et al. "Glibenclamide micro-particles", Seite 324
- P. H. LIST et al, "Arzneiformlehre", Wiss. Verlagsgesellschaft, Stuttgart, DE, 1985, S. 205-206

## Beschreibung

Glibenclamid, ein Sulfonylharnstoffderivat der Formel 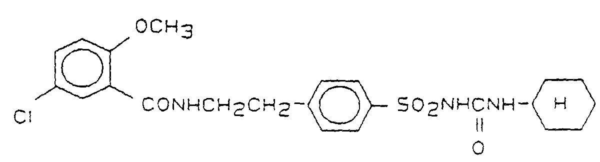 ist ein orales Antidiabetikum, das besonders zur Behandlung von Diabetes melitus geeignet ist (vgl. z.B. Deutsche Patentschrift 1 301 812).

Es ist bekannt, daß bei der geringen Löslichkeit von Glibenclamid in den Verdauungssäften die Resorptionsgeschwindigkeit vom Verteilungsgrad des Wirkstoffes abhängt (Deutsche Patentschrift 2348334 entsprechend US Patentschriften 3,979,520 und 4,060,634). Sehr geringe Teilchengrößen in der Größenordnung von weniger als 2 µ mit einer Oberfläche von mindestens 3 m²/g nach BET bieten den Vorteil einer hohen Resorptionsrate, die zu einem therapeutisch gewünschten, raschen Absinken des Blutzuckerspiegels führt.

Verschiedene Verfahren zur Herstellung von Glibenclamid mit hohen Oberflächen im Bereich von 3 bis 10 m²/g nach BET sind aus der Literatur bekannt. In der Deutschen Patentschrift 2348334 werden Methoden beschrieben zur Mikrofällung von Glibenclamid ausgehend von Glibenclamid bzw. von Salzen des Glibenclamid.

Mikrofeines Glibenclamid wird entsprechend dem genannten Stand der Technik z.B. durch Ausfällen des Wirkstoffs aus einem mit Wasser mischbaren organischen Lösungsmittel erhalten. Hierbei wird Glibenclamid z.B. in Dimethylformamid gelöst und die Lösung unter starkem Rühren in Wasser oder Wasser/Säure Mischungen einlaufen gelassen. Zur besseren Vermischung wird vorgeschlagen, die Lösung direkt in den Rührkopf eines Ultra-Turrax Turbinenrührers laufen zu lassen bzw. zu pumpen.

Alternativ kann das Natriumsalz des Glibenclamid in einem Gemisch von Methanol und Wasser gelöst werden. Die Lösung wird nach Filtration in Wasser eindosiert, dem die zur Neutralisation des Natriumsalzes ausreichende Menge Säure zugesetzt ist. Zur guten Durchmischung wird ebenfalls ein Ultra-Turrax Turbinenrührer eingesetzt.

Als weitere Variante wird der Einsatz wäßriger Lösungen des Alkalisalzes des Glibenclamid vorgeschlagen. Nachteilig ist die geringe Löslichkeit der Alkalisalze in Wasser, so daß der Einsatz wäßriger Lösungen zur Mikrofällung kein brauchbares technisches Verfahren darstellt.

Eine weitere Möglichkeit der Mikronisierung besteht in der Feinmahlung von Glibenclamid. Zur Erzielung der gewünschten hohen Oberflächen von bis zu 10 m²/g ist eine mehrmalige Mahlung auf Strahlmühlen erforderlich, evt. auch mit Zusatz von Mahlhilfsmitteln.

In der Patentschrift RO 89 209 (vgl. Chemical Abstracts 107, 28393g (1987)) wird eine andere Variantezur Herstellung von mikrofeinem Glibenclamid mit Teilchenvon 1-2 µ beschrieben. Glibenclamid wird in Dimethylformamid gelöst und die 80-85°C heiße Lösung zu vorgelegtem Wasser hinzugefügt bzw. das Wasser zur heißen Lösung gegeben.

Diese den Stand der Technik darstellenden Methoden besitzen unter verschiedenen Gesichtspunkten Nachteile in wirtschaftlicher Hinsicht, unter dem Aspekt der Qualität oder in Bezug auf den technischen Aufwand.

Bei der Mikrofällung gemäß Stand der Technik unter Verwendung organischer Lösemittel sind Nebenreaktionen des Glibenclamid mit den Lösemitteln nicht zu vermeiden. Die zulässigen Konzentrationen an Nebenprodukten in pharmazeutischen Wirkstoffen wie Glibenclamid sind sehr niedrig angesetzt. In der BP 80 ist der Gehalt an Nebenverbindungen wie dem Sulfonamid (4-[2-(5-Chlor-2-methoxybenzamido)-ethyl]-phenylsulfonamid) oder dem Urethan (4-[2-(5-Chlor-2-methoxybenzamido)-ethyl)phenylsulfonylcarbaminsäuremethylester) mit kleiner 0,4 % begrenzt. Dem Fachmann ist bekannt, daß beim Lösen von Glibenclamid in Dimethylformamid in Abhängigkeit von der Temperatur eine Rückspaltung zum Sulfonamid stattfindet, die in der Größenordnung von einigen Zehntelprozent bereits bei 40°-60°C erfolgt. Wird Glibenclamid in Methanol gelöst, wird bekanntermaßen das Urethan gebildet, das im Temperaturbereich von 40°-60°C im Zeitraum von wenigen Stunden Konzentrationen von einigen Zehntelprozent erreichen kann.

Da bei den für technische Verfahren erforderlichen Konzentrationen die Temperaturen bis zur vollständigen Lösung des Glibenclamids in DMF, Methanol oder Methanol/Wasser-Gemischen im Bereich von 40°-60°C liegen, ist eine Qualitätsverschlechterung des Glibenclamids bei diesen Fällverfahren auch bei sehr sorgfältiger Arbeitsweise nicht auszuschließen.

Ein weiterer Nachteil dieser Fällverfahren ist in der zusätzlichen wirtschaftlichen Belastung zu sehen, die durch die Entsorgung der org. Lösemittel entsteht, sei es durch Recyclisierung, sei es durch Verbrennung oder biologischen Abbau in der Kläranlage.

Der Restgehalt an Lösemitteln im Produkt ist bei diesen Verfahren zur Mikrofällung ein weiteres Problem. Besonders hochsiedene Lösemittel wie Dimethylformamid sind adsorptiv an der großen Oberfläche des Produktes gebunden. Eine weitgehende Entfernung, wie es für pharmazeutische Wirkstoffe erforderlich ist, ist möglich aber sehr aufwendig.

Weiteres ist dem Fachmann bekannt, daß die Oberflächen der mikrogefällten Produkte in weiten Bereichen schwanken können, da die Kristallwachstumsbedingungen durch die Fällbedingungen nicht ausreichend festgelegt sind. So wird häufig festgestellt, daß die Oberfläche des Wirkstoffs nicht im Rahmen der Spezifikation liegt. In solchen Fällen muß das Produkt unter hohen zusätzlichen Kosten umgearbeitet werden.

Die Mikrofällung aus Lösung wie auch die Feinmahlung stellen einen zusätzlichen Verfahrensschritt dar, der spezielle technische Einrichtungen erfordert.

Für die Feinmahlung ist eine Mahlanlage erforderlich, die üblicherweise aus einer Strahlmühle und einem aufwendigen Staubabscheidesystem besteht, um den pharmakologisch hochwirksamen Wirkstoff vollständig abzuscheiden.

Die Produktionsanlagen zur Mikrofällung erfordern spezielle Einrichtungen, um die vorzugsweise durchgeführte Vermischung der Lösungen in Rotor-Stator-Systemen wie dem Ultra-Turrax Turbinenrührer bzw. in Mischpumpen durchführen zu können.

Überraschender Weise wurde nun gefunden, daß die Mikrofällung auf technisch sehr einfache, ökonomisch und ökologisch sehr günstige Art und Weise durchgeführt werden kann.

Die Erfindung betrifft daher ein Verfahren zur Herstellung einer mikronisierten Form von Glibenclamid mit einer Oberfläche der Wirkstoffteilchen von mindestens 3 m²/g nach BET, dadurch gekennzeichnet, daß eine Suspension des schwerlöslichen Natriumsalzes des Glibenclamids in Wasser mit einer Säure angesäuert wird, der pH-Wert nach der Fällung des mikronisierten Glibenclamids bei 2,0 bis 4,5 liegt und die Temperatur bei der Fällung 30 bis 50°C beträgt, wobei mikrofein gefälltes Glibenclamid entsteht.

Besondere technische Einrichtungen sind zur Herstellung nicht erforderlich. Das erfindungsgemäße Verfahren kann in einem Standard-Rührkessel durchgeführt werden.

Ein weiterer Vorteil des erfindungsgemäßen Verfahrens liegt in der Tatsache, daß die Oberfläche des gefällten Wirkstoffes in reproduzierbarer Art und Weise durch die Fälltemperatur bestimmt werden kann.

Im Temperaturbereich von 20°-60°C ist z.B. bei der Umsetzung des Natriumsalzes die Oberfläche eine eindeutige Funktion der Temperatur. Bei 20°C werden im Mittel Oberflächen von 14-15 m²/g erreicht, bei 60°C eine Oberfläche von 2,5-3,0 m²/g. Die vorzugsweise gewünschte Oberfläche im Bereich von 5-10 m²/g wird im Temperaturintervall von 35-45°C erreicht.

Als Salze des Glibenclamids kommen in Frage die Salze der Alkalimetalle Lithium, Natrium oder Kalium und das Ammoniumsalz, vorzugsweise jedoch das Natriumsalz.

Zum Ausfällen kommen in Betracht die Mineralsäuren Salpetersäure, Schwefelsäure, Salzsäure oder Phosphorsäure und die organischen Säuren Essigsäure oder Zitronensäure Vorzugsweise wird Salpetersäure oder Salzsäure eingesetzt. Der pH-Wert der Suspension beträgt nach der Fällung 2,0 bis 4,5.

Durch Zusätze von oberflächenaktiven Stoffen läßt sich die kristalline Umwandlung der Glibenclamid-Salze zu Glibenclamid mikrofein beeinflussen. Als Netzmittel können physiologisch verträgliche ionogene und nicht ionogene Stoffe eingesetzt werden. Bevorzugt werden Polyoxyethylenstearate wie z.B. Polyoxyethylenstearat mit durchschnittlich 50 Ethylenoxid-Einheiten. Die Konzentration beträgt z.B. 0,05-1,0 Gew.-%, vorzugsweise 0,1-0,2 Gew.-% bezogen auf den Wirkstoff.

Mit Zusätzen von organischen mit Wasser mischbaren Lösemitteln wie z.B. Dimethylformamid oder niederen Alkoholen zu den Suspensionen läßt sich die Kristallgröße und damit die Oberfläche des Glibenclamids steuern.

Das nach dem erfindungsgemäßen Verfahren erhältliche Glibenclamid eignet sich zur Herstellung von Arzneimitteln.

Die erfindungsgemäße Herstellung von mirkonisiertem Glibenclamid mit einer Oberfläche von mindestens 3 m²/g, vorzugsweise 5-10 m²/g nach BET wird anhand der nachstehenden Beispiele erläutert:

### Beispiel 1

2480 l E-Wasser werden im Kessel vorgelegt und unter Rühren werden 62 kg Glibenclamid-Na-Salz eingetragen. Die Temperatur der Suspension wird auf 40°C eingestellt. Innerhalb von 30 min werden unter gutem Rühren 65,4 l 2n-Salpetersäure zulaufen gelassen. Das Glibenclamid fällt als fein verteilte Suspension aus, die abzentrifugiert und mit reichlich Wasser gewaschen wird. Nach dem Trocknen beträgt die Ausbeute 55,3 kg =̂ 96,5 % d.Th.. Das Produkt hat eine Oberfläche von 6,9 m²/g nach BET. Der Schmelzpunkt liegt bei 170°C.

### Beispiel 2

1500 ml Wasser werden vorgelegt. Darin werden erst 0,35 g Polyoxyethylenstearat 50 (®Macrogol) gelöst und anschließend 37,7 g Glibenclamid-Na-salz suspendiert. Die Temperatur der Suspension wird auf 30°C eingestellt. Innerhalb von 30 Min. laufen 40 ml 2 normale Salpetersäure unter gutem Rühren zu. Der pH-Wert der Suspension beträgt danach pH 2,2. Die Suspension von Glibenclamid mikrofein wird abgesaugt und sorgfältig mit Wasser gewaschen. Nach Trocknen im Vakuum bei 60°C beträgt die Ausbeute 33,97 =̂ 97,4 % d. Th.. Die Oberfläche nach BET liegt bei 8,7 m²/g.

### Beispiel 3

75,5 g Glibenclamid-Na-Salz werden in 2700 ml E-Wasser und 300 ml Methanol suspendiert. Bei 30°C werden unter Rühren innerhalb von 40 min. 80 ml 2n-salpetersäure zulaufen gelassen. Nach 1 stündigem Nachrühren wird das Produkt abgesaugt und mit Wasser reichlich gewaschen. Nach Trocknen im Vakuum bei 60°C erhält man eine Ausbeute von 67,8 g =̂ 96,8 % d. Th. an Glibenclamid mikrofein mti einer Oberfläche von 4,8 m²/g.

## Patentansprüche

1. Verfahren zur Herstellung einer mikronisierten Form von Glibenclamid der Formel 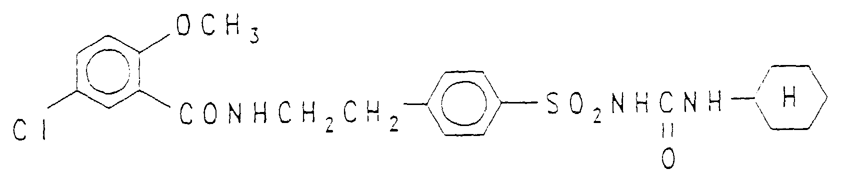 mit einer Oberfläche der Wirkstoffteilchen von mindestens 3 m²/g nach BET, dadurch gekennzeichnet, daß eine Susoension des schwerlöslichen Natriumsalzes des Glibenclamids in Wasser mit einer Säure angesäuert wird, der pH-Wert nach der Fällung des mikronisierten Glibenclamids bei 2,0 bis 4,5 liegt und die Temperatur bei der Fällung 30 bis 50°C beträgt, wobei mikrofein gefälltes Glibenclamid entsteht.

2. Verfahren nach Anspruch 1, dadurch gekennzeichnet, daß dem Wasser ein pharmakologisch verträgliches Netzmittel zugesetzt wird in einer Konzentration von 0,05 bis 1,0 Gew.-% bezogen auf den Wirkstoff.

3. Verfahren nach Anspruch 2, dadurch gekennzeichnet, daß dem Wasser ein Polyoxyethylenstearat, vorzugsweise Polyoxyethylenstearat mit durchschnittlich 50 Ethylenoxid-Einheiten, zugesetzt wird, vorzugsweise in einer Konzentration von 0,1 bis 0,2 Gew.-% bezogen auf den Wirkstoff.

4. Verfahren nach Anspruch 1, dadurch gekennzeichnet, daß dem Wasser organische, mit Wasser mischbare Lösemittel zugesetzt werden, wie z. B. Dimethylformamid oder niedere Alkohole.

5. Verfahren nach Anspruch 4, dadurch gekennzeichnet, daß eine Mischung von Wasser mit 5 - 30 Vol.-% Methanol zum Suspendieren des Glibenclamid-Na-Salzes eingesetzt wird.

## Claims

1. A process for the preparation of a micronized form of glibenclamide of the formula 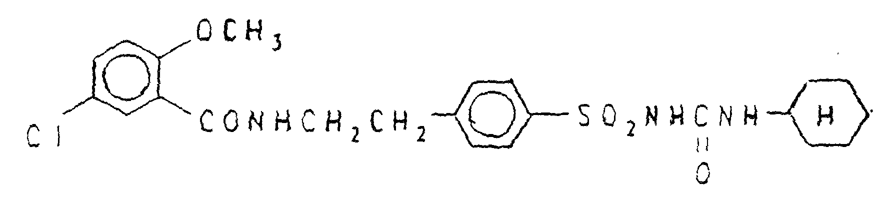 in which the active compound particles have a surface area of at least 3 m²/g by the BET method, which comprises acidifying a suspension of the sparingly-soluble sodium salt of glibenclamide in water using an acid, the pH after precipitation of the micronized glibenclamide being from 2.0 to 4.5 and the temperature during precipitation being from 30 to 50°C to give glibenclamide precipitated in microfine form.

2. The process as claimed in claim 1, wherein a pharmacologically acceptable wetting agent is added to the water in a concentration of from 0.05 to 1.0 % by weight, relative to the active compound.

3. The process as claimed in claim 2, wherein a polyoxyethylene stearate, preferably polyoxyethylene stearate containing an average of 50 ethylene oxide units, is added to the water, preferably in a concentration of from 0.1 to 0.2 % by weight, relative to the active compound.

4. The process as claimed in claim 1, wherein organic, water-miscible solvents, such as, for example, dimethylformamide or lower alcohols, are added to the water.

5. The process as claimed in claim 4, wherein a mixture of water with 5-30 % by volume of methanol is employed to suspend the Na salt of glibenclamide.

## Revendications

1. Procédé de préparation de glibenclamide de formule 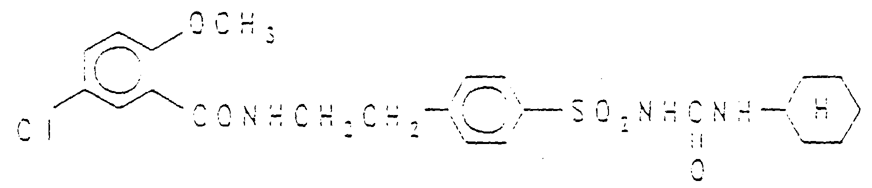 sous forme micronisée avec une surface de particules de principe actif d'au moins 3 m²/g selon la méthode BET, caractérisé en ce que l'on acidifie au moyen d'un acide une suspension du sel sodique du glibenclamide difficilement soluble dans l'eau, en ce que le pH après la précipitation du glibenclamide micronisé est de 2,0 à 4,5 et en ce que la température lors de la précipitation est de 30 à 50°C, grâce à quoi on obtient du glibenclamide précipité microfein.

2. Procédé selon la revendication 1, caractérisé en ce que l'on ajoute à l'eau un agent dispersant pharmacologiquement acceptable en une concentration de 0,05 à 1,0 % en poids par rapport au principe actif.

3. Procédé selon la revendication 2, caractérisé en ce que l'on ajoute à l'eau un stéarate de polyoxyéthylène, de préférence un stéarate de polyoxyéthylène ayant en moyenne 50 motifs oxyde d'éthylène, de préférence en une concentration de 0,1 à 0,2 % en poids par rapport au principe actif.

4. Procédé selon la revendication 1, caractérisé en ce que l'on ajoute à l'eau des solvants organiques miscibles à l'eau tels que par exemple du diméthylformamide ou des alcools inférieurs.

5. Procédé selon la revendication 4, caractérisé en ce que l'on utilise un mélange d'eau avec 5 à 30 % en volume de méthanol pour mettre le sel sodique de glibenclamide en suspension.
